# EUROPEAN PATENT SPECIFICATION

(11) **EP 0 456 252 B1**
(45) Date of publication and mention of the grant of the patent: **24.04.1996**
(21) Application number: 91107611.5
(22) Date of filing: 10.05.1991
(51) Int. Cl.: G01N 33/50, G01N 33/579, C12Q 1/37

(54) **Method for determination of pyrogen content**
Verfahren zum Nachweis des Pyrogen-Gehaltes
Procédé pour la détermination de la teneur de pyrogène

(30) Priority: 11.05.1990 JP 122679/90; 30.11.1990 JP 337562/90
(43) Date of publication of application: 13.11.1991
(73) Proprietor: TANABE SEIYAKU CO., LTD., Chuo-ku Osaka (JP); DAICEL CHEMICAL INDUSTRIES, LTD., Sakai-shi Osaka-fu 590 (JP)
(72) Inventor: Tosa, Tetsuya, Kyoto-shi, Kyoto-fu (JP); Sato, Tadashi, Ibaraki-shi, Osaka-fu (JP); Watanabe, Taizo, Nagaokakyo-shi, Kyoto-fu (JP); Minobe, Satoshi, Otsu-shi, Shiga-ken (JP); Nawata, Masahiro, Osaka-fu (JP); Nagamatsu, Shinji, Himeji-shi, Hyogo-ken (JP)
(74) Representative: Hansen, Bernd, Dr. Dipl.-Chem.

(56) References cited:
- EP-A- 0 228 666
- EP-A- 0 350 004
- EP-A- 0 418 517
- GB-A- 2 092 470
- US-A- 4 491 660
- WPIL, Week 8202, Derwent Publications Ltd., London (GB); AN 82-03166E/
- WPIL, Week 8736, Derwent Publications Ltd., London (GB); AN 87-254437/

## Description

### FIELD OF THE INVENTION

The present invention relates to a method for determination of a pyrogen content. More particularly, it relates to a method for determination of a pyrogen content having excellent sensitivity and operating characteristics, which is applicable to even a specimen which has hitherto been difficult to determine because of the presence of determination interfering substances and the like.

### BACKGROUND OF THE INVENTION

Pyrogens are pyrogenetic substances which abnormally raise the body temperature of a homothermic animal in a very small amount. When a pyrogen is introduced into the human body, for example, by intravenous injection of a medicine contaminated with it, apart from the main activity of the medicine, the pyrogen causes severe fever. It is said that, when this action of pyrogen becomes serious, it causes severe fever accompanied with chill and shudder and, occaisionally, death from a shock. Many substances such as bacterial substances, inflammatory substances, plant polysaccharides, blood group substances and the like have been known as pyrogens. Among them, bacterial substances have the most important influence on fever and are called as bacterial toxins. In general, bacterial toxins are classified into exotoxins and endotoxins. Particularly, it is said that an endotoxin acting as so-called O-antigen the main component of which is a cell wall lipopolysaccharide (LPS) of a gram negative bacterium has the strongest pyrogenicity.

Accordingly, it is very important to detect or determine a pyrogen content, for example, to prevent contamination of a pyrogen during the production of medicines.

As a method for detection or determination of a pyrogen content, for example, fever test using a rabbit, or Limulus test using a blood cell extract of Limulus polyphenus have been known. Particularly, Limulus test has been often used from viewpoints of sensitivity, simplicity, quantitative properties and the like.

However, Limulus test is liable to be interfered or activated by various substances present in a determination system and, therefore, complicated pre-treatment is required or, sometimes, it becomes difficult to determine a pyrogen content depending upon a specimen. Further, there are some substances other than pyrogens which are positive in Limulus test and they interfere with precise determination of an pyrogen content. In order to solve this problem, the use of highly purified reagents has been proposed. However, such reagents are very expensive. Further, it is considered to be difficult to determine a very small amount of a pyrogen contained in a substance having a low solubility.

Recently, in order to detect an endotoxin which is one of pyrogens, a method which comprises bringing a pyrogen-free activated charcoal obtained by treatment with an acid into contact with a specimen and reacting the activated charcoal with Limulus lysate to carry out Limulus test easily and rapidly has been proposed (Japanese Patent Laid Open Publication No. 152425/1981). However, this method is yet unsatisfactory from viewpoints of specificity, sensitivity and quantitative properties for pyrogens. Further, U.S. Patent No. 4,491,660 discloses that a certain polymer which can absorb an endotoxin can be used for concentrating and detecting the endotoxin. However, this polymer is yet unsatisfactory from viewpoints of specificity, sensitivity, quantitative properties and the like.

EP-A-0 350 004 discloses a method for determining the pyrogen content in which a specimen is brought into contact with an adsorbent composed of a nitrogen-containing heterocyclic compound bonded to a water-insoluble carrier directly or through a spacer. After, or without, eluting the pyrogen adsorbed on the adsorbent, the content of the adsorbed pyrogen is determined by means of a Limulus method.

JP-A-62-176560 discloses a filter of a centrifugal separator which consists of a cylindrical sample cup having a filter at the bottom and a filtrate cup connected to the bottom of the sample cup.

### OBJECTS OF THE INVENTION

The present inventors have intensively studied to develop a method for determination of a pyrogen content having excellent specificity, sensitivity and quantitative properties for pyrogens. As a result, it has been found that, by using an adsorbent having pyrogen adsorbability and a centrifugation type filtration unit, the determination of a pyrogen content by Limulus test can be readily and simply carried out in high specificity and high sensitivity even in the presence of various interfering substances or other Limulus test positive substances. Further, by using certain solid adsorbents, a method for determination of a pyrogen content which can solve problems in conventional methods using adsorbents such as activated charcoal and the like has been realized.

Namely, the main object of the invention is to provide a method for determination of a pyrogen content having excellent specificity, sensitivity and quantitative properties for pyrogens, which is applicable to even a specimen which has hitherto been difficult to determine by a conventional Limulus test because of the presence of determination interfering substances and the like.

This object as well as other objects and advantages of the present invention become apparent to those skilled in the art from the following description with reference to the accompanying drawings.

### BRIEF EXPLANATION OF DRAWINGS

Figs. 1 to 3 are schematic cross sections of examples of the centrifugation type filtration unit used in the method of the present invention, respectively.

Figs. 4 to 10 illustrate examples of the calibration curve used in the method of the present invention, respectively.

### SUMMARY OF THE INVENTION

According to the present invention, there is provided a method for determining the pyrogen content of a liquid specimen using a filtration unit comprising a filtrate cup and a specimen cup removably attachable to the opening part of the filtrate cup, said specimen cup communicating with the filtrate cup through a filtration membrane, said method comprising the steps of:
contacting the liquid specimen in the specimen cup with a solid adsorbent capable of selectively absorbing pyrogen,
filtering non-adsorbed material through the filtration membrane under conditions for accelerating the permeation through the membrane to remove the non-adsorbed material into the filtrate cup and
reacting pyrogen adsorbed on the adsorbent retained in the specimen cup with a detecting reagent to determine its pyrogen content. Hereinafter, this method is referred to as the filtration unit method.

### DETAILED DESCRIPTION OF THE INVENTION

In the filtration unit method, normally, permeation is accelerated by applying a centrifugal force as a driving force (centrifugal filtration). Further, permeation can also be accelerated by applying a force other than a centrifugal force such as pressure (e.g. pressure filtration, filtration under reduced pressure, etc.) or the like as a driving force. Furthermore, when a specimen solution is retained with a hydrophobic membrane and brought into contact with an adsorbent, permeation can also be accelerated by addition of an alcohol or the like. Thus, the expression "under conditions for accelerating premeation through the membrane" used herein means these operations.

The expression such as "a centrifugation type filtration unit" or "a centrifugation type filtration unit for centrifugal filtration comprising a filtrate cup and a specimen cup removably attached to the opening part of the filtrate cup" used herein merely means that the device is suitable for centrifugation, and the present invention is not limited to centrifugation. Filtration other than centrifugal filtration can also be employed in the present invention.

Even if the filtration unit is not used, the determination of a pyrogen content can be carried out. However, by using the filtration unit, non-adsorbed materials can be removed readily, rapidly, completely and precisely and, further, the reaction with a detecting reagent, for example, Limulus reagent can be carried out in situ in the specimen cup. Accordingly, operating characteristics and precision of the determination are remarkably improved in comparison with the determination without using the unit.

The centrifugation type filtration unit used in the present invention comprises a filtrate cup and a specimen cup removably attached to the opening part of the filtrate cup by a suitable means, for example, fitting, clamp, screw and the like. The specimen cup is communicated with the filtrate cup through a filtration membrane. The shape and size of the filtrate cup and the specimen cup are not specifically limited in so far as they are made of materials which do not interfere with the determination of a pyrogen content, for example, glass, synthetic resins, metal and the like.

The membrane used for the centrifugation type filtration unit is that having a function such that it retains a solution in the specimen cup under normal conditions and, upon centrifugation, the solution permeates through it. Normally, an ultrafiltration membrane (UF membrane) is preferred. However, the present invention is not limited to an ultrafiltration membrane, and any membrane having the above function can be used. For example, a hydrophobic microfiltration membrane (MF membrane) can be used. As the ultrafiltration membrane, for example, that having a cutoff molecular weight of 10,000 to 3,000,000 can be appropriately selected. When the cutoff molecular weight is too small, the filtration rate becomes too slow. On the other hand, when the cutoff molecular weight is too large, leakage of a solution is caused. The material of the membrane is not specifically limited in so far as it does not interfere with the determination of a pyrogen content. For example, the ultrafiltration membrane made of cellulose, cellulose acetate, cellulose triacetate, polyelectrolyte conjugated cellulose, polysulfone, vinyl/acryl copoplymer, polyether sulfone or the like can be used.

As the MF membrane, for example, that having a pore opening of 0.01 to 10 µm can be appropriately selected and the material thereof is not specifically limited in so far as it does not interfere with the determination of a pyrogen content. For example, the MF membrane made of polytetrafuluoroethylene, polyvinylidene difluoride or the like can be used. Further, these membranes can also be used as a double membrane in combination with a hydrophobic membrane made of, for example, polypropylene or the like.

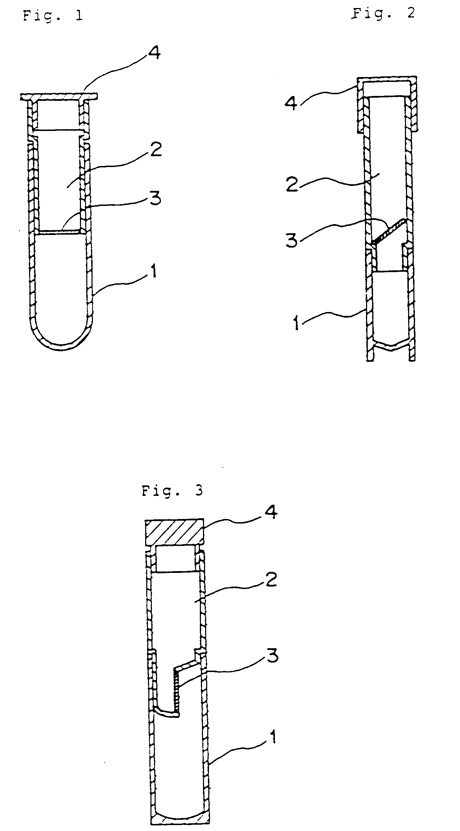

Figs. 1 to 3 are schematic cross sections of examples of the centrifugation type filtration unit used in the present invention, respectively.

The unit shown in Fig. 1 comprises a filtrate cup (1) and a specimen cup (2) removably attached to the opening part of the filtrate cup by engagement and, at the bottom of the specimen cup (2), a filtration membrane (3) is placed so that a solution of a specimen is transferred to the filtrate cup through the filtration membrane (3) by centrifugation. The specimen cup (2) has a lid (4). The unit shown in Fig. 2 is fundamentally the same as that of Fig. 1 except that the filtration membrane (3) is inclined at the bottom of the specimen cup. The unit of Fig. 3 is also fundamentally the same as that of Fig. 1 except that a part of the bottom of the specimen cup (2) is extruded and the filtration membrane (3) is placed perpendicular to the axial direction. These centrifugation type filtration units are known and, for example, they can be commercially available under the trade name of ULTRAFREE-CL (sold by Japan Millipore Limited), ULTRACENT (manufactured by Toso Kabushiki Kaisha, Japan), CENTRICUT (manufactured by Kurabo, Japan) and the like.

In the filtration unit method, the adsorbent to be used may be that being capable of selectively adsorbing a pyrogen, compared with coexisting materials in a specimen. The adsorbents explained hereinafter are representative examples of the preferred adsorbents. However, the present invention is not limited thereto and a person skilled in the art will readily find out other adsorbents suitable for the filtration unit method.

Examples of these adsorbents include those disclosed in Japanese Patent Laid Open Publication No. 183412/1982. Such an adsorbent is composed of a nitrogen-containing cyclic compound bound to a water-insoluble carrier (base material) directly or through a spacer (hereinafter referred to as the "heterocyclic type adsorbent").

Examples of the heterocyclic type adsorbent include those wherein the nitrogen-containing heterocyclic compound is represented by the general formula:

R-A-X (I)

wherein R is a heterocyclic group having nitrogen atom as the hetero atom; A is a single bond, an alkylene group or an alkenylene group; X is hydrogen atom or a functional group when A is a single bond, or a functional group when A is an alkylene group or an alkenylene group; and the heterocyclic group and the alkylene group may be substituted with one or more substituents,

the water-insoluble carrier is that having hydroxy, amino, carboxyl or a halogen atom and

the spacer is a compound of the formula:

NH₂(CH₂)ₙNH₂,

NH₂(CH₂)ₙCOOH,

NH₂(CH₂)ₙOH,

or

HOOC(CH₂)ₙCOOH

wherein n is an integer of 1 to 12.

Examples of the nitrogen-containing heterocyclic compound of the formula [I] include those wherein R is a nitrogen-containing heterocyclic group having, for example, imidazole nucleus, pyrazole nucleus, pyrimidine nucleus, pyridazine nucleus, pyrazine nucleus, purine nucleus, acridine nucleus, triazole nucleus, oxadiazol nucleus, tetrazole nucleus, indazole nucleus, benzotriazole nucleus, benzopyridazole nucleus, benzopyrimidine nucleus, benzopyrazine nucleus, naphthylidine nucleus or the like; A is a single bond and X is hydrogen atom or a functional group such as amino, hydroxy, carboxyl or the like, or A is an alkylene group having 1 to 12 carbon atoms such as methylene, ethylene, propylene, butylene, hexylene, octylene, decamethylene or dodecamethylene, or an alkenylene group having 2 to 12 carbon atoms such as vinylene, propenylene, butenylene, hexenylene or octenylene and X is a functional group such as amino, hydroxy, carboxyl or the like. The nitrogen-containing heterocyclic group of R and the alkylene group of A may be optionally substituted with one or more substituents (e.g. carboxyl, oxo, C₁₋₆ alkyl, hydroxy, amino, C₁₋₆ alkoxy, etc.). Preferred examples of the nitrogen-containing heterocyclic compounds are those of the formula [I] wherein R is a nitrogen-containing heterocyclic group having imidazole nucleus, pyrimidine nucleus, purine nucleus or acridine nucleus, A is a single bond, ethylene or ethylene substituted with carboxyl, and X is amino, carboxyl or hydroxy.

In the present invention, any water-insoluble carrier which can be bound to the nitrogen-containing heterocyclic compound of the general formula [I] directly or through a spacer can be used. Representative examples of the water-insoluble carrier include those having hydroxy, amino, carboxyl or a halogen atom. Preferred examples of the water-insoluble carrier having hydroxy are polysaccharides such as cellulose, agarose, cross-linked dextran and the like, hydroxylated polystyrene resins (e.g., hydroxyalkylated styrene-divinylbenzene copolymer, etc.), polyvinyl alcohol and the like. Examples of the water-insoluble carrier having amino are aminoalkylated polysaccharides (e.g., amioalkylated cellulose such as aminoetylcellulose or aminohexylcellulose, aminoalkylated agarose such as aminohexylagarose, p-aminobenzylcellulose, p-aminobenzyl-agarose, etc.), chitosan, aminoalkylated polystyrene resins (e.g., aminoalkylated styrene-divinylbenzene copolymer, etc.), polyacrylamides, aminoalkylated polyacrylamide (e.g. aminoethyl-polyacrylamide, etc.), aminoalkylated porous glass (e.g. aminopropyl porous glass, etc.) and the like. Examples of the water-insoluble carrier having carboxyl are carboxylated polysaccharides (e.g. carboxy-alkylated agarose such as carboxyhexylagarose or carboxypentylagarose, carboxyalkylated cellulose such as carboxymethylcellulose, carboxylated crosslinked dextran such as carboxymethyl-crosslinked dextran, etc.), carboxyalkylated polyacrylamide (e.g., carboxymethylpolyacrylamide, etc.), carboxylic acid resins (e.g., acrylic acid-divinylbenzene copolymer, etc.) and the like. Examples of the water-insoluble carrier having a halogen atom are halogenoalkylpolystyrene resins (e.g., chloromethylated styrene-divinylbenzene copolymer, etc.) and the like.

Preferred examples of the above heterocyclic type adsorbent used in the present invention are those obtained by bonding the nitrogen-containing heterocyclic compound [I] such as histidine, histamine, urocanic acid, uracil, orotic acid, cytosine, 5-methylcytosine, 2-amino-4,6-dimethylpyrimidine, 2-amino-4-hydroxy-6-methylpyrimidine, adenine or 6,9-diamino-2-ethoxyacridine to the water-insoluble polysaccharide through alkylenediamine as the spacer by using, at least, either one of a monoepoxide (e.g. epichlorohydrin, etc.) and an aliphatic dialdehyde (e.g. glutaraldehyde, etc.).

Further, in the present invention, the adsorbent other than the heterocyclic type adsorbent as described above can also be used. Examples of such an adsorbent include a solid adsorbent composed of an aliphatic nitrogen-containing compound bound to the base material directly or through a spacer (hereinafter referred to as an aliphatic type adsorbent). This is also one example of the adsorbents.

The compound which constitutes the aliphatic type adsorbent has no nitrogen-containing heterocycle and aromatic amino group but contains an aliphatic nitrogen atom. Normally, this compound has a functional chain composed of 3 to 50 atoms, particularly, 3 to 30 atoms. The number of chain-constituting atoms (chain length) is the number of atoms which constitutes a continuous chain member in the longest chain. For example, in the case of the functional chain of CH₂CH(OH)CH₂NH(CH₂)₆NH₂, the chain length is the sum of the intermediate chain (substituted propylene) (3) and diaminohexylene (8), i.e., 11. Nitrogen atom, oxygen atom (ether) and sulfur atom (thioether) are counted as the chain-constituting atoms in addition to carbon atom.

Nitrogen atom should be bound to an aliphatic carbon atom, for example, carbon atom of methylene. As the aliphatic nitrogen atom, for example, the nitrogen atom of primary amino, secondary amino, tertiary amino, quaternary ammonium, imino or the like can be used. Particularly, those of primary amino (NH₂) and secondary amino (NH) are preferred. The aliphatic nitrogen atom may be any of termial-constituting atoms of a compound and intermediate-constituting atoms of a chain. Further, it may be a pendant nitrogen atom bound to an intermediate atom directly or through other aliphatic carbon atoms. As the functional chain, those containing primary or secondary amino aliphatic nitrogen atom and a chain having 3 or more carbon atoms adjacent to the nitrogen atom are preferred. In the case of the functional chain as exemplified above, hexylene group (and hydroxy-substituted propylene group) is adjacent to two aliphatic nitrogen atoms.

Examples of the functional group are shown by the following A to D.
Aliphatic chain A: -CH₂CH(OH)CH₂NHR¹

Examples of R¹ include hydrogen atom and the following groups (the compound in the brackets is R¹NH₂ or R¹OH corresponding to the group R¹).
(CH₂)ₙNH₂ (N = 1 to 12) [alkylenediamine], COCH(NH₂)(CH₂)₄NH₂ [lysine], COCH(NH₂)(CH₂)₃NH₂ [ornithine]
Aliphatic chain B: -CH₂CH(OH)CH₂NH(CH₂)₅NHR
Examples of R: (CH₂)ₙNH₂ (n = 1 to 12), (CH₂)ₙNHC(=NH)₂ (n = 1 to 12)
Aliphatic chain C: -CH₂CH(OH)CH₂NHCOCH₂CH₂NHR³
Examples of R³: (CH₂)ₙNH₂ (n = 1 to 12), (CH₂)ₙNHC(=NH)₂ (n = 1 to 12).
Aliphatic chain D: -CH₂CH(OH)CH₂NH(CH₂)₆NHR⁴
Example of R⁴: C(=NH)NH₂, COCH(NH₂)(CH₂)₄NH₂, (CH₂)ₙNH₂ (n = 1 to 12)

The base material is normally a porous material and, hereinafter, the explanation is mainly directed to a porous base material. However, the base material is not specifically limited thereto and any base material which has an adsorption capacity necessary for the quantitative analysis.

The porous base material having pore size of 50 nm to 1 µm is preferred. The base material has a structure which can fix the aliphatic chain having nitrogen atom directly or indirectly, and examples thereof include a material having an active site (e.g., active hydrogen) which can react with a functional group such as hydroxy, amino or the like and other substances.

Examples of the base material are those similar to the water-insoluble carrier of the heterocyclic type adsorbent. Preferred examples include polysaccharides (including derivatives thereof such as aminoalkylated polysaccharides, carboxyalkylated polysaccharides, etc.; polysaccharides such as cellulose, crosslinked dextran, chitosan, etc.), organic synthetic polymers (e.g., polyacrylonitrile, polysulfone, polyamide, polyvinyl alcohol, polystyrene, polyacrylic acid, aminoalkylated or halogenoalkylated polystyrene, polyacrylamide, etc.) and inorganic polymers (e.g., silica gel, alumina, aminopropyl porous glass, various ceramics, etc.). Further, a material obtained by suitably introducing hydroxy, amino or the like which is useful for bonding an aliphatic chain into the above porous material according to a conventional method can also be used as the base material.

In the production of the aliphatic type adsorbent, examples of the means for bonding the aliphatic chain having aliphatic nitrogen atom to the above porous base material directly or through a spacer include covalent bonding, ionic bonding, hydrophobic bonding, coordinate bonding and the like. For example, the means described in Japanese Patent Laid Open Publication No. 183712/1982 can be employed. The aliphatic type adsorbent can be produced, for example, by activating the porous base material such as cellulose, etc. with the epoxy compound such as epichlorohydrin, etc. and then bonding the aliphatic chain having amino, hyroxy, thiol, carboxyl or the like to it, or by activating the base material, introducing a suitable spacer into it and then reacting with the aliphatic chain having amino, carboxyl, aldehyde, thiol or the like.

Among the porous adsorbents obtained as described above, most preferred adsorbents are, for example, those using polysaccharide such as cellulose, etc. as the base material and having the aliphatic chain represented by the above formula -CH₂CH(OH)CH₂NHR¹ or -CH₂CH(OH)CH₂NH(CH₂)₅NHR¹ and the pore opening of 100 to 500 nm.

Thus, the filtration unit method can be carried out by bringing a specimen into contact with the adsorbent in the specimen cup of the filtration unit, separating a non-adsorbed material by centrifugation to transfer it into the filtrate cup, optionally, washing and centrifuging the adsorbent in the specimen cup to transfer the washing into the filtrate cup, discarding the contents of the filtrate cup to remove substances other than pyrogens, and then reacting the adsorbent which has adsorbed pyrogens in the specimen cup with a detecting reagent, for example, Limulus reagent to determine the pyrogen content.

Normally, the determination of a pyrogen content can be rapidly, readily and precisely carried out by, after completion of Limulus reaction, separating the reaction solution from the adsorbent by centrifugation to transfer it into the filtrate cup to carry out a quantitative operation.

Hereinafter, detailed explanation of the quantitative operation is set forth.

In the method of the present invention, the specimen to be examined should be used in a solution state, for example, a solution containing water or an aqueous solution and, particularly, an aqueous solution is preferred. Further, since the adsorbent used in the present invention absorbs neither determination interfering substances of Limulus test, for example, amino acids (e.g., L-cysteine, etc.), antibiotics (e.g., penicillin G, streptomycine, etc.), protein denaturants (e.g., urea, etc.), serine protease inhibitors (e.g., diisopropylfluorophosphoric acid, etc), saccharides (e.g. glucose, etc.), sugar alcohols, sodium chloride, Ringer's solution and the like, nor Limulus test positive substances such as dextran, (1-3)- β-D-glucan and the like, the method of the present invention can also be applied to a specimen containing these substances which have hitherto been difficult to determine the pyrogen content. In addition, according to the present invention, endotoxins contained in a protein solution, blood plasma or blood serum can be determined.

The adsorption operation is carried out in the specimen cup and operating conditions can be appropriately selected according to a particular specimen. Normally, a pyrogen can be specifically and efficiently adsorbed by using 3 to 270 mg, preferably 7 to 180 mg of the adsorbent per 1 ml of the specimen and carrying out the adsorption under conditions of a temperature of 4 to 40°C , pH of 4 to 8, preferably, 6 to 7 and an ionic strength of not more than 0.2. When the adsorption operation is carried out under these conditions for about 15 minutes or longer, the pyrogen in the specimen is almost completely adsorbed on the adsorbent and the pyrogen is concentrated on the adsorbent. Further, re-adsorption can also be carried out after completion of the adsorption, thereby, sensitivity of determination can be further raised.

The adsorbent which has adsorbed the pyrogen is separated from the specimen according to a known method and then, washed with pyrogen-free water, a pyrogen-free buffer solution or the like to remove substances other than pyrogens.

In the present invention, the adsorbent thus washed can be directly subjected to Limulus test as it is.

In general, Limulus test is classified into synthetic substrate methods and gelation methods and the synthetic substrate methods are further classified into colorimetry and fluorimetry according to a particular kind of substrates. In the present invention, any of them can be employed. For example, the adsorbent wherein the pyrogen has been adsorbed is reacted with Limulus lysate and a synthetic substrate having a chromophore or a fluorophore at 25 to 40°C, usually, 37°C for 10 to 120 minutes. After completion of the reaction, the reaction mixture is subjected to colorimetry or fluorimetry and then the pyrogen content in the specimen is determined according to a calibration curve which is prepared separately according to the same manner as described above.

The synthetic substrate is not specifically limited and is appropriately selected from those which are normally employed in this kind of reaction.

In the case of colorimetry, the determination can be carried out by using a synthetic substrate, for example, Ac-Ile-Glu-Ala-Arg-pNA or Boc-Leu-Gly-Arg-pNA (wherein Ac is acetyl, Boc is t-butoxycarbonyl and pNA is p-nitroaniline) and measuring the absorbance at the wavelength of 405 nm. In the case of fluorimetry, the determination can be carried out by using a synthetic substrate, for example, Boc-Leu-Gly-Arg-MCA (wherein Boc is the same as defined above and MCA is 7-amino-4-methylcumarin) and measuring the fluorescence at the excitation wavelength of 380 nm and fluorescent wavelength of 460 nm.

In the case of the gelation method, Limulus lysate is added to the adsorbent wherein the pyrogen has been adsorbed and then, immediately the reaction is started, the adsorbent is removed or after standing at a low temperature for a while to proceed the reaction and the reaction is further continued at 25 to 40°C, normally, at 37°C for 10 to 120 minutes. Turbidity of the reaction solution due to gelation is measured with time, and then, the concentration of the pyrogen in the specimen can be determined based on a calibration curve which is prepared separately according to the same manner as described above.

Hereinabove, the method using Limulus reagent as the detecting reagent has been set forth. However, depending upon a particular kind of specimen, the quantitative operation can be appropriately carried out using other available detecting reagents. In the present invention, concentration of the pyrogen and separation of the pyrogen from determination interfering substances or detection reaction positive substances are carried out by using adsorbents having pyrogen adsorbability and, therefore, there are some cases where a detecting reagent having the same efficiency as that of Limulus reagent is not required.

The adsorbent used in the present invention can be produced, for example, according to the same manner as that described in the above Japanese Patent Laid Open Publication No. 183712/1982.

Detailed explanation of PS method is set forth below for illustrative purposes.

According to PS method, the determination of a pyrogen content can be carried out by bringing a specimen solution into contact with a solid adsorbent (aliphatic type adsorbent) composed of a nitrogen-containing aliphatic compound bonded to a base material directly or through a spacer and, after solid-liquid separation, reacting the solid adsorbent with a pyrogen detecting reagent.

The explanation of the aliphatic type adsorbent has been already set forth with respect to the filtration unit method. The aliphatic type adsorbent is capable of selectively adsorbing a pyrogen and, therefore, by carrying out solid-liquid separation (including washing step) after contacting with a specimen solution, the pyrogen is removed from other substances present in the specimen solution.

The solid adsorbent is reacted with a pyrogen detecting reagent to determine the pyrogen content. In this case, for example, when turbidimetric Limulus test is employed, a supernatant is formed within a short period of time (within about 10 seconds) due to precipitation of the solid adsorbent and luminous flux through a test tube for measurement is not shut off and, therefore, the gelation time can be measured without separating the solid adsorbent from the specimen solution. Thus, the method for determination of a pyrogen content which has specificity and sensitivity as well as quantitative properties can be realized by using the adsorbent having suitable selective adsorptivity and precipitability and separating the pyrogen present in the specimen solution from determination interfering substances and the like. For the determination based on gelation, although an inversion method is also known, it is preferred to employ turbidimetric technique having excellent quantitative properties.

As a matter of course, in PS method, the filtration unit as explained above can also be used. When the filtration unit is used, excellent operating characteristics in solid-liquid separation (including washing step) can be utilized in PS method.

According to the present invention, sensitivity of Limulus test can be remarkably improved and the pyrogen content of a specimen even containing interfering substances which has hitherto been considered to be difficult can be determined. Further, even if Limulus test positive substances other than pyrogens are contained in the specimen, separation and determination of pyrogens can be carried out readily and precisely. Furthermore, it is possible to use a cheaper reagent instead of Limulus reagent. Thus, the method for determination of a pyrogen content of the present invention is suitable for check of the production steps of medicines and the like.

The following Examples and Reference Examples further illustrate preferred aspects of the present invention in detail.

### Example 1

### Study on calibration curve

To pyrogen-free water (2 ml) was added a predetermined amount of endotoxin EC-5 (FDA reference endotoxin) to prepare an aqueous EC-5 solution (concentration of EC-5: 0, 0.05, 0.10 or 0.20 EU/ml; EU is endotoxin unit).

This solution was placed in a specimen cup of a centrifugation type filtration unit as shown in Fig. 1 in which a suspension of the adsorbent prepared in Reference Example 1 described hereinafter in water [0.3 g (wet weight)/ ml, 100 µl] was contained, and the mixture was stirred at 50 r.p.m. at 20 to 25°C for 60 minutes using a round mixer. Then, the mixture was centrifuged at 2,300 G for 15 minutes and a filtrate cup is removed to discard the contents. The filtrate cup was put back and 25 mM NaCl solution (0.5 ml) was added to the specimen cup, followed by further centrifuging for 10 minutes. Likewise, the contents in the filtrate cup were discarded and, again, the filtrate cup was attached to the specimen cup. To the specimen cup was added pyrogen-free water (2 ml) and the mixture was centrifuged for 25 minutes and then the contents in the filtrate cup were discarded.

Then, to the specimen cup was added Limulus amebocyte lysate [prepared by dissolving 1 vial (for 0.2 ml, sensitivity: FDA reference endotoxin EC-2 concentration was gelled at a concentration of 0.1 ng/ml) of Limulus single test Wako (manufactured by Wako Pure Chemical Industries, Ltd., Japan) into 0.4 M Tris hydrochloride buffer (pH 8.0, 1.5 ml) containing 0.04 M magnesium chloride] (50 µl) at 4°C and the mixture was gently stirred by a mixer. To this was added 0.6 mM aqueous Boc-Leu-Gly-Arg-MCA solution (200 µl) as a substrate solution which had been heated to 70°C, and the mixture was gently mixed and then reacted at 37°C for 25 to 30 minutes. After completion of the reaction, 12.5 % aqueous acetic acid solution (2.3 ml) was added to terminate the reaction and the filtrate cup was attached thereto and then the mixture was centrifuged at 2,300 G for 30 minutes.

Then, fluorimetry was carried out at 380 nm of excitation wavelength and 460 nm of fluorescent wavelength.

The results are shown in Table 1.

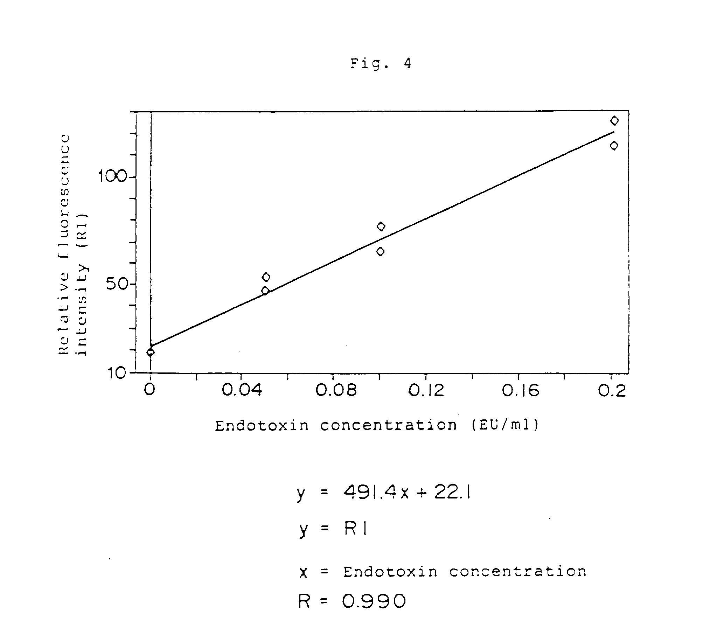

The data obtained were plotted on a graph wherein the ordinate indicated the relative intensity (RI) in fluorimetry and the abscissa indicated LPS concentration to obtain a calibration curve as shown in Fig. 4.

As seen from Fig. 4, the calibration curve shows good linear relationship.

**Table 1**

| Run No. | Endotoxin concentration (EU/ml) | RI |
|---|---|---|
| 1 | 0.0 | 18.8 |
| 2 | 0.0 | 19.9 |
| 3 | 0.05 | 47.1 |
| 4 | 0.05 | 53.9 |
| 5 | 0.10 | 65.2 |
| 6 | 0.10 | 78.8 |
| 7 | 0.20 | 125.7 |
| 8 | 0.20 | 113.4 |

### Example 2

### Study on calibration curve

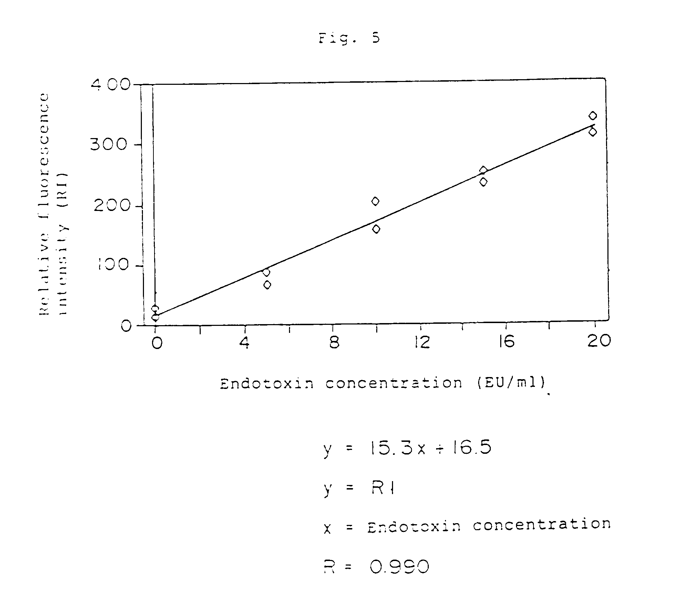

To pyrogen-free water (2 ml) was added a predetermined amount of LPS (derived from E. coli 0128: B12) to prepare an aqueous LPS solution (concentration of LPS: 0, 5, 10, 15 or 20 pg/ml). According to the same manner as that described in Example 1, LPS content in the LPS solution was determined. As a result, the calibration curve as shown in Fig. 5 which had good linear relationship was obtained.

### Example 3

### Determination of endotoxinin phenylalanine

To a 2 % aqueous L-phenylalanine solution containing no endotoxin was added endotoxin EC-5 (0.1 EU/ml). Then, by using the calibration curve obtained in Example 1, determination of the endotoxin content was repeated twice according to the same manner as that described in Example 1. The adsorbent was washed in turn with 0.02 M NaCl (2 ml) and water (2 ml) after centrifugation of non-adsorbed substances.

The average value of the results of the determination was 0.114 EU/ml (recovery: 114 %).

On the other hand, the endotoxin content in the above EC-5 containing 2% L-phenylalanin solution was determend by using Limulus reagent having gelation sensitivity of 0.03 EU/ml (reagent A) or Limulus reagent having that of 0.14 EU/ml (reagent B). As a negative control, endotoxin-free distilled water was used. As a positive control, EC-5 aqueous solution of which EC-5 concentration was 0.033 EU/ml in the case of the reagent A, or EC-5 aqueous solution of which EC-5 concentration was 0.167 EU/ml in the case of the reagent B was used. As a result, both the reagents A and B gave negative results with respect to the negative control and positive results with the positive control. However, the endotoxin content in the phenylalanine solution could not be detected.

### Example 4

### Determination of endotoxin in amino acid alimentation solution

To an endotoxin-free amino acid alimentation solution (Amizet 10, manufactured by Tanabe Seiyaku Co., Ltd., Japan) was added endotoxin EC-5 (0.2 EU/ml) and the mixture was diluted 4 times with endotoxin-free water. According to the same manner as that described in Example 3, the endotoxin determination was carried out. The average value of the results of the determination was 0.066 EU/ml (recovery: 132 %).

On the other hand, according to the same manner as that described in Example 3, the endotoxin content was determined by using reagent A, reagent B, negative control and positive control except that endotoxin-added amino acid alimentation solution was diluted 6 times in the case of the reagent A or the solution was not diluted in the case of the reagent B. As a result, the endotoxin content in the amino acid alimentation solution could not determined.

### Example 5

### Determination of endotoxin in penicillin G

To an aqueous endotoxin-free potassium penicillin G solution (50000 U/ml) was added endotoxin EC-5 (0.5 EU/ml) and the mixture was diluted 10 times with endotoxin-free water. According to the same manner as that described in Example 3, the endotoxin content was determined. As a result, the average of the results of the determination was 0.062 EU/ml (recovery: 124 %).

On the other hand, according to the same manner as that described in Example 3, endotoxin content was determined by using reagent A, reagent B, negative control and positive control except that endotoxin-added potassium penicillin G solution was diluted 15 times in the case of the reagent A or diluted 3 times in the case of the reagent B. As a result, although the endotoxin content in the aqueous potassium penicillin G solution could be determined in the case of the reagent B, in the case of the reagent A, the endotoxin content could not determined.

### Example 6

### Study on calibration curve

To pyrogen-free water (100 µl) was added a predetermined amount of endotoxin EC-5 to prepare an aqueous EC-5 solution (concentration of EC-5: 0, 0.2, 0.4, 0.8 or 1.6 EU/ml).

This solution (100 µl) was mixed with a 10 % suspension (pH 6.0, µ= 0.02, 900 µl) of the adsorbent prepared in Reference Example 1 described hereinafter and the mixture was placed in a centrifugation type filtration unit as shown in Fig. 1 wherein a double membrane composed of a MF membrane (polyvinylidene difluoride) and a hydrophobic membrane (polypropylene) were used as a filtration membrane and stirred at 50 r.p.m. at room temperature for 15 minutes using a round mixer. Then, it was centrifuged at 1700 G for 5 minutes and the filtrate cup was removed to discard the contents. The filtrate cup was put back and endotoxin-free 0.02 M NaCl solution (1 ml) was added to the specimen cup, followed by stirring with a mixer for 20 seconds and further centrifugation at 1700 G for 5 minutes. The contents in the filtrate cup were discarded and then to the specimen cup was added Limulus amebocyte lysate reagent QLC-1000 (300 µl) manufactured by Daiichi Kagaku Yakuhin K.K. [prepared by mixing a solution obtained by dissolving Limulus amebocyte lysate (1 vial) in endotoxin-free 0.1 M Tris hydrochloride buffer (0.01 M MgCl₂, pH 8.0, 1.4 ml) and a solution obtained by dissolving Ac-Ile-Glu-Ala-Arg-pNA into endotoxin-free distilled water (6.5 ml) in the mixing ratio of 1 : 2] at 4°C. The mixture was stirred for 15 seconds by a mixer and then reacted at 37°C for 30 minutes. After completion of the reaction, 25% aqueous acetic acid solution (200 µl) was added to terminate the reaction. The filtrate cup was attached thereto and the mixture was centrifuged at 1700 G for 5 minutes, and then, the absorbance at 405 nm of the reaction solution transferred to the filtrate cup was measured.

The results are shown in Table 2.

**Table 2**

| Run No. | Endotoxin concentration (EU/ml) | Absorbance |
|---|---|---|
| 1 | 0.0 | 0.128 |
| | | |
| 2 | 0.2 | 0.260 |
| 3 | 0.4 | 0.449 |
| 4 | 0.8 | 0.921 |
| 5 | 1.6 | 1.613 |

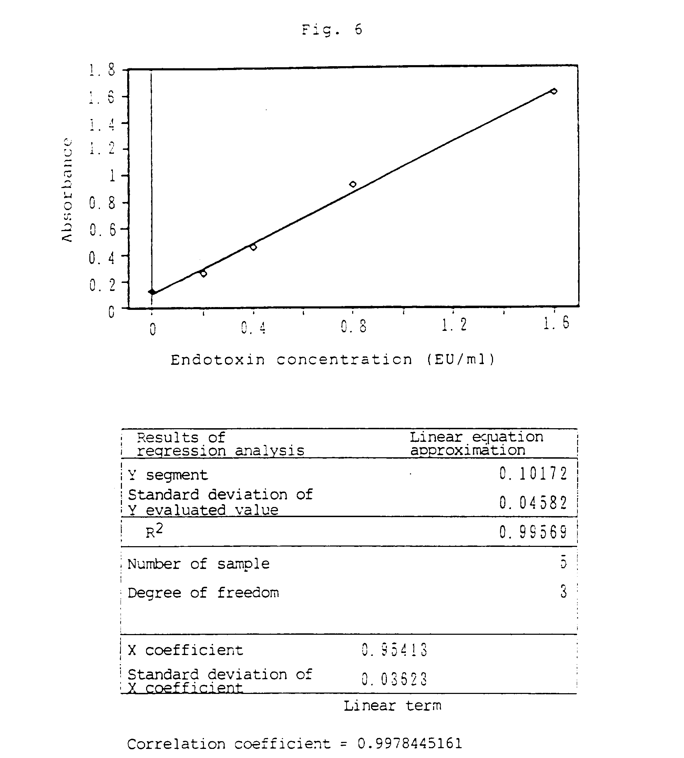

The data obtained were plotted on a graph wherein the ordinate indicated the absorbance and the abscissa indicated endotoxin concentration to obtain the calibration curve as shown in Fig. 6.

As seen from Fig. 6, the calibration curve shows good linear relationship.

### Examples 7 to 11

### Determination of endotoxin in various materials

According to the same manner as that described in Example 6, the endotixin content was determined except that endotoxin (EC-5) was added to an aqueous solution of endotoxin-free sodium chloride, glucose, cysteine hydrochloride, potassium penicillin G, β-1,3-D-glucan, respectively, and the calibration curve obtained in Example 6 was used.

The results are shown in Table 3.

**Table 3**

| Run No. | Specimen solution | Amount of endotoxin (EU/ml) | Endotoxin content (EU/ml) |
|---|---|---|---|
| 7 (1) | 0.15 M aqueous sodium chloride solution | 0.5 | 0.52 |
| 7 (2) | 0.5 M aqueous sodium chloride solution | 0.5 | 0.52 |
| 7 (3) | 1.0 M aqueous sodium chloride solution | 0.5 | 0.51 |
| 7 (4) | 2.0 M aqueous sodium chloride solution | 0.5 | 0.42 |
| 8 | 50 % aqueous glucose solution | 0.5 | 0.55 |
| 9 | aqueous cysteine hydrochloride solution | 0.5 | 0.50 |
| 10 | aqueous potassium penicillin G solution | 0.5 | 0.58 |
| 11 | aqueous β-1,3-D-glucan solution | 0.5 | 0.55 |

### Example 12

### Determination of endotoxin in human serum albumin (HSA)

To a 5 % HSA solution was added endotoxin (EC-5) (0 or 0.5 EU/ml) and, according to the same manner as that described in Example 6, the endotoxin content was determined using the calibration curve obtained in Example 6 except for using an adsorbent suspension of pH 5.5, µ= 0.1.

The results are shown in Table 4.

**Table 4**

| Amount of endotoxin (EU/ml) | Endotoxin content (EU/ml) |
|---|---|
| 0 | 0.11 |
| 0.5 | 0.60 |

### Example 13

### Study on calibration curve

According to the same maner as that described in Example 6, the results as shown in Table 5 were obtained except that the concentration of endotoxin (EC-5) solution was 0, 0.05, 0.1, 0.2 or 0.4 (EU/ml) and the enzyme reaction time was 40 minutes.

**Table 5**

| Run No. | Endotoxin concentration (EU/ml) | Absorbance |
|---|---|---|
| 1 | 0 | 0.364 |
| 2 | 0.05 | 0.441 |
| 3 | 0.1 | 0.572 |
| 4 | 0.2 | 0.855 |
| 5 | 0.4 | 1.503 |

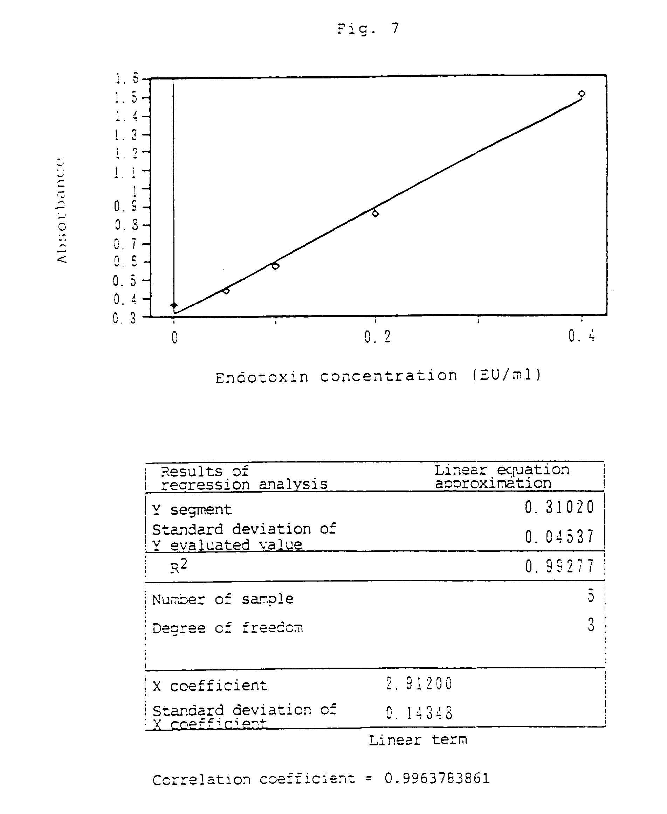

The data obtained were plotted on a graph wherein the ordinate indicated the absorbance and the abscissa indicated the endotoxin concentration to obtain the calibration curve as shown in Fig. 7.

As seen from Fig. 7, the calibration curve shows good relationship.

### Examples 14 to 16

### Determination of endotoxin in various materials

According to the same manner as that described in Example 7, the endotoxin content was determined except that endotoxin (EC-5) was added to an aqueous solution of endotoxin-free phenylalanine, methionine and amino acid alimentation solution, respectively and the calibration curve obtained in Example 7 described above was used.

The results are shown in Table 6.

**Table 6**

| Run No. | Speciment solution | Amount of endotoxin (EU/ml) | Endotoxin content (EU/ml) |
|---|---|---|---|
| 14 | 2 % aqueous phenylalanine solution | 0.1 | 0.10 |
| 15 | 5 % aqueous methionine solution | 0.1 | 0.10 |
| 16 | amino acid alimentation solution | 0.2 | 0.20 |

### Example 17

### Study on calibration curve (turbidimetric technigue)

To pyrogen-free water (2 ml) was added a predetermined amount of endotoxin EC-5 to prepare an aqueous EC-5 solution (concentration of EC-5: 0.05, 0.1, 1 or 10 EU/ml).

This solution (200 µl) and a 10 % suspension (pH 6.0, µ= 0.02, 900 µl) of the adsorbent prepared in Reference Example 1 described hereinafter were placed in a centrifugal type filtration unit wherein a double membrane composed of a MF membrane (polyvinylidene difluoride) and a hydrophobic membrane (polypropylene) were used as a filtration membrane and stirred at 50 r.p.m. at room temperature for 15 minutes using a round mixer. Then, it was centrifuged at 1700 G for 5 minutes and the filtrate cup was removed to discard the content. Then, to the specimen cup were added Limulus amebocyte lysate [prepared by dissolving 1 vial of Limulus single test Wako (manufactured by Wako Pure Chemical Industries, Ltd., Japan) into endotoxin-free 0.1 M Tris hydrochloride buffer (pH 7.3, 5 ml)] (200 µl) and pyrogen-free distilled water (100 µl) at 4°C. The mixture was stirred for 15 seconds by a mixer and then allowed to stand at 4°C for 15 minutes. Then, the filtrate obtained by centrifugation at 1700 G for 5 minutes was transferred to a pyrogen-free test tube having a cap and the gelation time was measured with Toxinometer (turbidimetric technique).

On the other hand, a predetermined concentration of an aqueous EC-5 solution (concentration of EC-5: 0.05, 0.1, 1 or 10 EU/ml) was prepared and the gelation time of the filtrate obtained by treating the above aqueous EC-5 solution (200 µl) and Limulus amebocyte lysate reagent (200 µl) as desribed above without any pyrogen adsorption operation by an adsorbent was measured (conventional method).

The results are shown in Table 7. According to the method of the present invention, almost the same value as that measured by the conventional method was obtained.

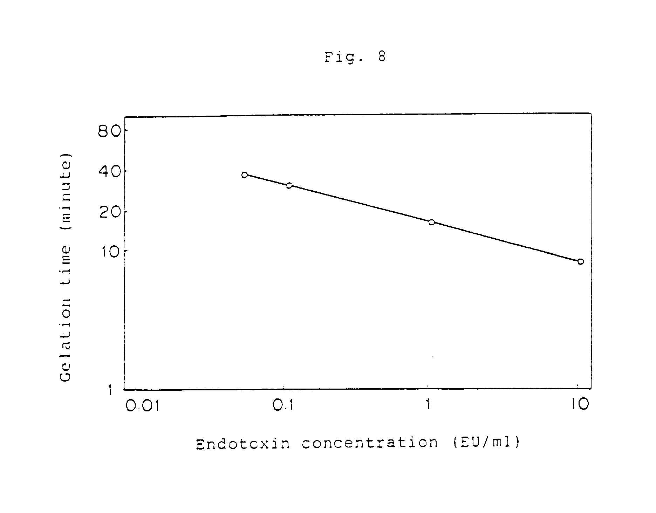

The data obtained were plotted on a graph wherein the ordinate indicated the gelation time and the abscissa indicated the endotoxin concentration to obtain the calibration curve as shown in Fig. 8.

As seen from Fig. 8, the calibration curve shows good relationship.

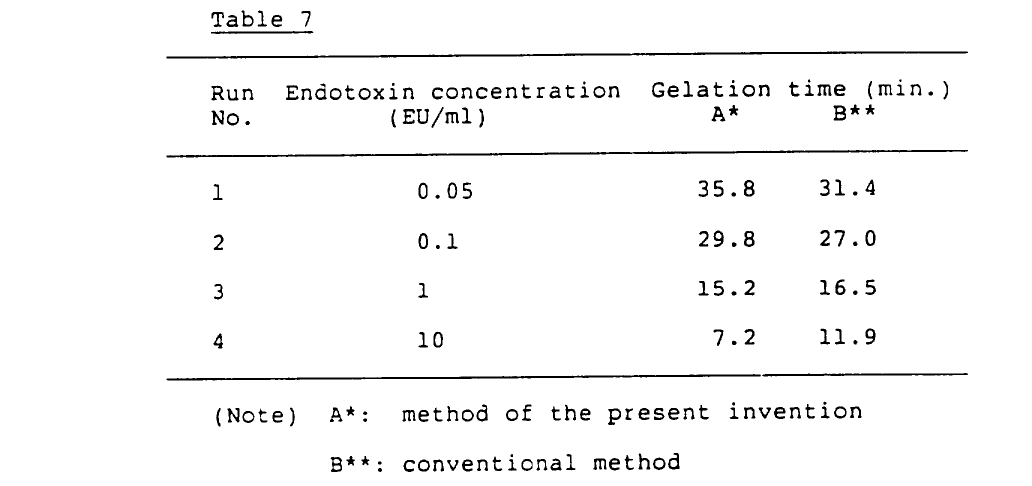

### Example 18

To pyrogen-free water (PFW) was added a pyrogen (manufactured by DIFCO, derived from E. coli 0111: B4) to prepare an aqueous pyrogen solution of various pyrogen concentration C₀ (pg/ml). The concentration of the pyrogen was calculated from the calibration curve prepared by using the above pyrogen with Toxinometer (turbidimetric technique).

An aliphatic type adsorbent, A-1 type (300 mg, wet weight) was placed in a pyrogen-free (PF) test tube having a cap and an aqueous pyrogen solution (3 ml) was added and, immediately, the mixture was stirred for 15 minutes by a round mixer (25 r.p.m., 20 to 25°C). After completion of the adsorption treatment, the solution was filtered by a pyrogen-free MF filter unit. The adsorbent on the filter was collected into a PF test tube with a PF spatula and to this was added pyrogen-free water to obtain a 30 % suspension.

To the bottom of a test tube for Toxinometer was added a suspension (100 µl) which was allowed to stand at 4°C. Then, a LAL test solution (ES test Wako) dissolved at 4°C (100 µl) was added. After the mixtue was reacted with the Limulus reagent for 10 minutes, the test tube was warmed in an incubator maintained at 37°C for 10 seconds and the gelation time T_{g} (minute) was measured with the toximater. The pyrogen content determined based on the gelation time is shown as the value C (pg/ml) per 1 ml of the undiluted solution in Table 8 in addition to C₀, T_{g} and C/C₀.

As a result, almost constant C/C₀ is obtained and logarithmic C shows good linear relationship with logarithmic T_{g}. Accordingly, when the gelation time of a specimen solution is measured according to the same manner, the pyrogen content can be determined based on the above calibration curve.

**Table 8**

| C₀ | T_{g} | C | C/C₀ |
|---|---|---|---|
| 0.5 | 80.0 | 0.1 | 0.20 |
| 0.5 | 75.2 | 0.1 | 0.20 |
| 3.1 | 44.6 | 0.4 | 0.13 |
| 3.1 | 45.6 | 0.5 | 0.16 |
| 6.3 | 31.0 | 1.2 | 0.19 |
| 6.3 | 28.4 | 1.4 | 0.22 |
| 21.9 | 19.6 | 4.7 | 0.21 |
| 21.9 | 19.2 | 4.9 | 0.22 |
| 86.4 | 14.2 | 14 | 0.16 |
| 86.4 | 12.6 | 20 | 0.23 |

### Example 19

### Determination of endotoxin in rabbit serum

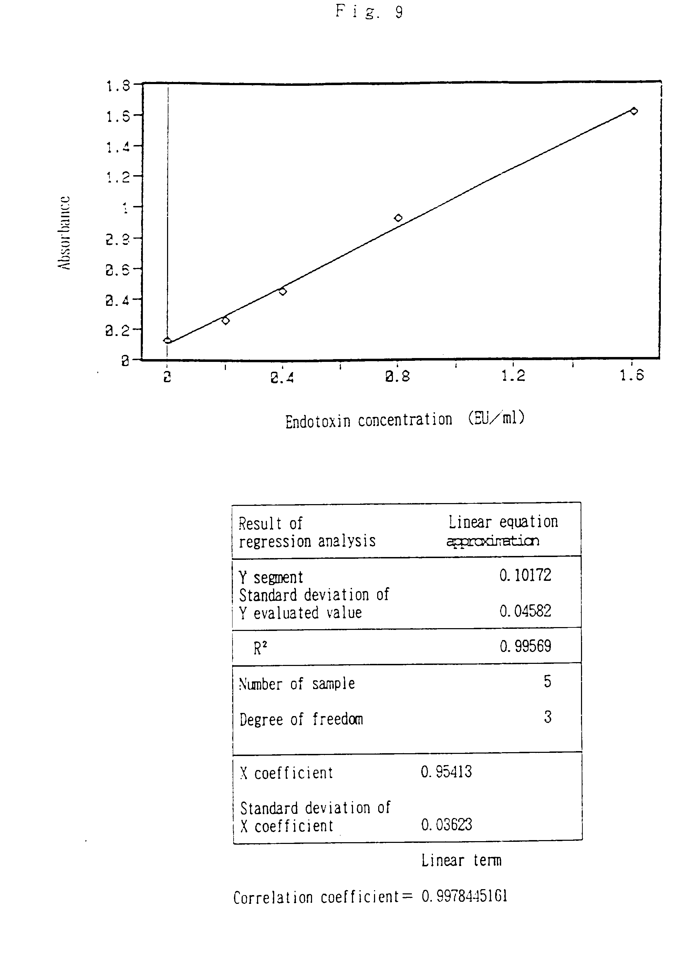

To rabbit serum was added endotoxin (EC-5) (0 or 10 EU/ml). This solution was diluted 10 times with endotoxin-free acetate buffer (pH5.5). This solution (100 µl) was mixted with a 10 % suspension (900 µl; suspended in the same buffer as above) of the adsorbent prepared in Reference Example 1 described hereinafter and the mixture was placed in a centrifugation type filtration unit described in Example 6 and stirred at 4°C for 30 minutes. Then, it was centrifuged at 1700 G for 5 minutes and the filtrate cup was removed to discard the contents. The filtrate cup was put back and endotoxin-free 0.02 M NaCl solution (1 ml) was added to the specimen cup, followed by stirring with a mixer for 20 seconds and further centrifugation at 1700 G for 5 minutes. The contents in the filtrate cup were discarded and the specimen cup was heated at 70°C for 10 minutes. Then, to the specimen cup was added Limulus amebocyte lysate reagent (the same as Example 6, 300 µl) at 4°C. The mixture was stirred for 15 seconds and then reacted at 37°C for 30 minutes. After completion of the reaction, 25 % aqueous acetic acid solution (200 µl) was added to terminate the reaction. The filtrate cup was attached thereto and the mixture was centrifuged for 5 minutes. Then, the absorbance at 405 nm of the reaction solution transferred to the filtrate cup was measured, and the endotoxin content in rabbit serum was calculated by using a calibration curve (shown in Fig. 9). The results are shown in Table 9.

Concomitantly, the calibration curve was obtained in the same manner as described above except that pyrogen-free distilled water was used instead of rabitt serum and the heat-treatment at 70°C was not carried out.

**Table 9**

| Amount of endotoxin (EU/ml) | Endotoxin Content (EU/ml) |
|---|---|
| 0 | 1.32 |
| 10 | 12.45 |

### Example 20

### Determination of endotoxin in human plasma

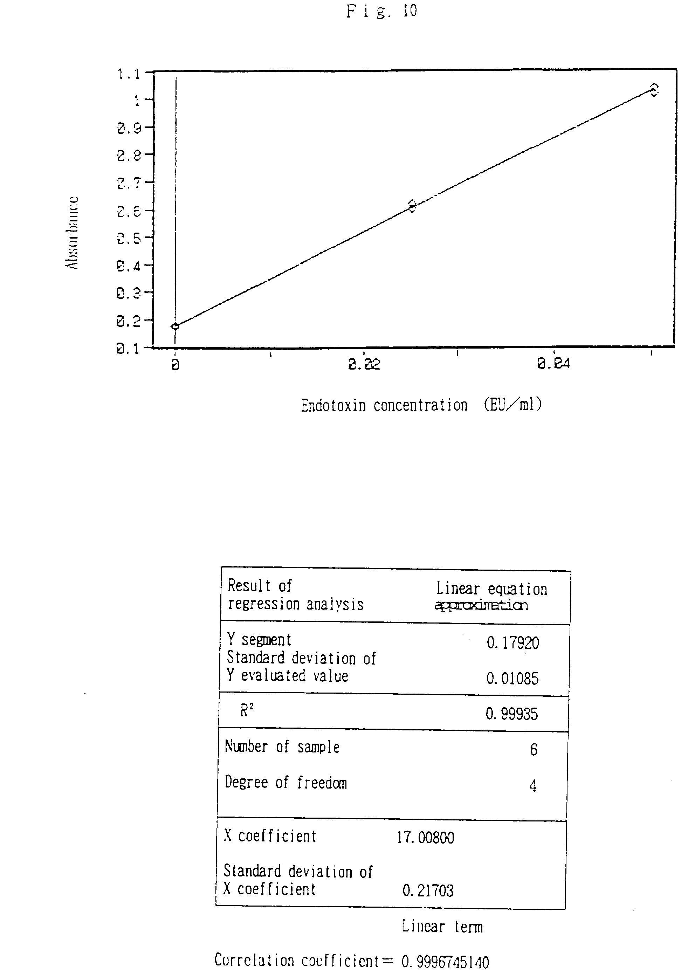

To human plasma was added endotoxin (EC-5) (0 or 1 EU/ml). This solution was diluted 40 times with endotoxin-free distilled water. This solution (400 µl) was added into a centrifugation type filtration unit described in Example 6 and heated at 65°C for 2 hours. Then, to this solution was added a 15 % suspension (600 µl; suspended in endotoxin-free acetate buffer (pH 5.5)) of the adsorbent prepared in Reference Example 1 described hereinafter and the mixture was stirred at room temperature for 15 minutes. Then, it was centrifuged for 5 minutes and the filtrate cup was removed to discard the content. The filtrate cup was put back and endotoxin-free 0.02 M NaCl solution (1 ml) was added to the specimen cup, followed by stirring with a mixer for 20 seconds and further centrifugation for 5 minutes. The contents in the filtrate cup were discarded and then to the specimen cup was added Limulus amebocyte lysate reagent (the same as Example 6, 300 µl) at 4°C. The mixture was stirred and then reacted at 37°C for 40 minutes. After completion of the reaction, the reaction mixture was treated in the same manner as described in Example 19 and the absorbance at 405 nm of the reaction solution was measured. The endotoxin content in human plasma was calculated by using a calibration curve (shown in Fig. 10). The results are shown in Table 10.

Concomitantly, the calibration curve was obtained in the same manner as described above except that pyrogen-free distilled water was used instead of human plasma and the heat-treatment at 65°C was not carried out.

**Table 10**

| No. | Amount of endotoxin (EU/ml) | Endotoxin content (EU/ml) |
|---|---|---|
| 1 | 0 | 0.04 |
| | 1 | 0.98 |
| | | |
| 2 | 0 | 0 |
| | 1 | 1.17 |
| | | |
| 3 | 0 | 0.01 |
| | 1 | 0.89 |
| | | |
| 4 | 0 | 0 |
| | 1 | 1.00 |

### Production of heterocyclic type adsorbent

### Reference Example 1

(1) Sepharose CL-4B (trade name of an agarose derivative manufactured by Pharamacia Fine Chemicals, 350 g, wet weight) was suspended in a distilled water (500 ml). 2 N Aqueous sodium hydroxide (200 ml) and epichlorohydrin (50 ml) were added and the mixure was stirred at 40°C for 2 hours. After completion of the reaction, the mixture was filtered and the residue was washed with distilled water to obtain epichlorohydrin-activated Sepharose CL-4B. Epichlorohydrin-activated Sepharose CL-B thus obtained was suspended in a 0.6 % aqueous solution of hexamethylenediamine (1.4 liters) heated to 60°C and the suspension was stirred at 60°C for 2 hours. After completion of the reaction, the mixture was filtered and the residue was washed with distilled water to obtain 3-(6-aminohexylamino)-2-hydroxy-propylated Sepharose CL-4B (370 g, wet weight). When the aminohexyl content of the resulting Sepharose was measured by titration, it was about 37.6 µmol/g (wet weight).
(2) 3-(6-aminohexylamino)-2-hydroxy-propylated Sepharose CL-4B (370 g, wet weight) was suspended in 4 N aqueous sodium hydroxide (700 ml). Epichlorohydrin (700 ml) was added to the suspension at 65°C and the mixture was stirred. After completion of the reaction, water was added to the mixture to cool to 50°C or lower. Then, the mixture was filtered, and the residue was washed with a distilled water to give epichlorohydrin-activated 3-(6-aminohexylamino)-2-hydroxy-propylated Sepharose CL-4B. This was suspended in an aqueous solution of 20 % histidine hydrochloride hydrate (adjusting to pH 12 with an aqueous sodium hydroxide, 2.1 liters) heated to 90°C and the suspension was stirred at 80 to 90°C for 30 minutes. After completion of the reaction, the mixture was filtered and the residue was thoroughly washed with a distilled water. The residue was suspended in a distilled water (1 liter) and subjected to aultoclaving at 120°C for 20 minutes and then filtered. The residue was thoroughly washed in turn with 0.2 N aqueous hydrochloric acid, 0.2 N aqueous sodium hydroxide, 1.5 N aqueous sodium chloride and distilled water. Thus, water-insoluble adsorbent containing agarose as a carrier, histidine as a ligand and hexamethylenediamine as a spacer center (390 g, wet weight) was obtained. The histidine content per 1 g (wet weight) of the adsorbent was 20.4 µmol.

### Reference Example 2

According to the same manner as that described in Reference Example 1, an adsorbent was obtained except that Cellulofine GCL-2000-m (trade name of a cellulose derivative manufactured by Chisso Co., Ltd., Japan, 350 g) was used in place of Sepharose CL-4B. The resulting adsorbent was the water-insoluble adsorbent containing cellulose as a carrier, histidine as a ligand and hexamethylenediamine as a spacer center (340 g, wet weight). The histidine content per 1 g (wet weight) of the adsorbent was 58 µmol.

### Reference Example 3

3-(6-aminohexylamino)-2-hydroxy-propylated Sepharose CL-4B (18 g, wet weight) prepared according to the same manner as that described in Reference Example 1 (1) was supended in 0.05 M phosphate buffer (pH 7.0, 45.6 ml). To the suspension was added 25 % aqueous glutaraldehide solution (19.2 ml) and the mixture was stirred at room temperature for 2 hours. After completion of the reaction, the mixture was filtered and the residue was thoroughly washed with 0.1 M phosphate buffer (pH 7.0) to obtain glutaraldehide-activated 3-(6-aminohexylamino)-2-hydroxy-propylated Sepharose CL-4B. This was suspended in 15 mM histamine-0.1 M phosphate buffer (pH 7.0, 59.6 ml) and the suspension was stirred at room temperature for 2 hours. After completion of the reaction, the mixture was filtered, and the residue was washed with a 1 M aqueous solution of sodium chloride (600 ml). The residue was suspended in 0.1 M phospahte buffer (pH 7.0, 30 ml). To the suspension was added sodium borohydride (0.3 g) and the mixture was stirred at room temperature for one hour. After completion of the reaction, the mixture was filtered and the residue was thoroughly washed in turn with 1 M aqueous sodium chloride solution and distilled water. Thus, water-insoluble adsorbent containing agarose as a carrier, histamine as a ligand and hexamethylenediamine as a spacer center (20.7 g, wet weight) was obtained. The histamine content per 1 g (wet weight) of the adsorbent was 4.1 µmol.

### Reference Example 4

### Production of aliphatic type adsorbent

### A-1 type:

Beads of porous cellulose gel carrier (a) (50 g, wet weight) was dehydrated by filtration under reduced pressure after washing with water and dispersed in 0.6 N aqueous sodium hydroxide solution (110 ml). To the mixture was added epichlorohydrine (16 ml) which was reacted at 60°C for 30 minutes. To the epoxy-activated intermediate (b) which had been filtered and washed with water was added a ligand solution and the mixture was reacted at 70°C for one hour. The ligand solution was prepared by dissolving 65 % hexamethylenediamine (4 ml) in water (100 ml). The reaction solution was filtered, washed with water, adjusted to pH 10.4 with hydrochloric acid, washed with water and then dehydrated to obtain about 55 g of a wet A-1 type adsorbent (c) (See Table 11).

**Table 11**

| g (wet weight)/g (dry weight) |
|---|
| (a) 17.0 [1.82 ml/g (wet weight)] |
| (b) 15.8 [epoxy content: 174 µmol/g (dry weight)] |
| (c) 18.2 [1.45 ml/g (wet weight)] |
| [hexamethylenediamine content: 133 µmol/g (dry weight) (elemental analysis)] |

This separating agent (adsorbent) has CH₂CH(OH)CH₂NH(CH₂)₆NH₂ (chain length: 11) as its main functional chain.

According to the same manner, a separating agent having the hexamethylenediamine content of 50 to 600 can be obtained by changing the reaction conditions. It is considered that the separating agent obtained by changing the reaction conditions may have both CH₂CH(OH)CH₂NH(CH₂)₆NHCH₂CH(OH)CH₂ (chain length: 14) and CH₂CH(OH)CH₂NH(CH₂)₆NH₂ (chain length: 11) as the main functional chain.

### A-2 type:

This is obtained by a condensation reaction of A-1 type adsorbent with lysine.

Functional chain:

CH₂CH(OH)CH₂NH(CH₂)₆NHCOCH(NH₂)(CH₂)₄NH₂

### B-1 type:

This is obtained by using a porous cellulose gel as a base material and adding epichlorohydrin and ammonia thereto. The resultant has CH₂CH(OH)CH₂NH₂ and CH₂CH(OH)CH₂NHCH₂(OH)CH₂ as the functional chain and is in the form of beads having the particle diameter of about 50 to 200 µm and the size of the largest pore is about 100 to 500 nm.

### B-2 type:

This is obtained by a condensation reaction of B-1 type adsorbent with lysine.
Functional chain: CH₂CH(OH)CH₂NHCOCH(NH₂)(CH₂)₄NH₂

## Claims

1. A method for determining the pyrogen content of a liquid specimen using a filtration unit comprising a filtrate cup and a specimen cup removably attachable to the opening part of the filtrate cup, said specimen cup communicating with the filtrate cup through a filtration membrane, said method comprising the steps of:
contacting the liquid specimen in the specimen cup with a solid adsorbent capable of selectively adsorbing pyrogen,
filtering non-adsorbed material through the filtration membrane under conditions for accelerating the permeation through the membrane to remove the non-adsorbed material into the filtrate cup, and
reacting pyrogen adsorbed on the adsorbent retained in the specimen cup with a detecting reagent to determine its pyrogen content.

2. A method according to claim 1, wherein, after completion of a reaction with Limulus lysate as the detecting reagent in the presence of a synthetic substrate, the adsorbent is separated from the reaction solution through the filtration membrane by centrifugation to transfer the reaction solution to the filtrate cup and the pyrogen content of the reaction solution determined.

3. A method according to claim 1, wherein the filtration membrane is an ultrafiltration membrane having a cutoff molecular weight of 10,000 to 3,000,000, or a microfiltration membrane having a pore opening of 0.01 to 10 µm.

4. A method according to claim 1, wherein the adsorbent is a heterocyclic type adsorbent composed of a nitrogen containing cyclic compound of the formula:
R-A-X
wherein R is a nitrogen containing heterocyclic group, A is a single bond, an alkylene group having 1 to 12 carbon atoms or an alkenylene group having 2 to 12 carbon atoms, and X is hydrogen, amino, hydroxy or carboxyl, when A is a single bond, or X is amino, hydroxy or carboxyl, when A is an alkylene or alkenylene group, said heterocyclic group and alkylene group being optionally substituted with one or more groups selected from carboxyl, oxo, an alkyl group having 1 to 6 carbon atoms, hydroxy, amino and an alkoxy group having 1 to 6 carbon atoms; and
a water-insoluble carrier having hydroxy, amino carboxyl or a halogen atom;
said nitrogen containing cyclic compound being bound to said water-insoluble carrier directly or through a spacer compound selected from:
NH₂(CH₂)ₙNH₂,
NH₂(CH₂)ₙCOOH,
NH₂(CH₂)ₙOH
and
HOOC(CH₂)ₙCOOH
wherein n is an integer of 1 to 12.

5. A method according to claim 4, wherein the nitrogen containing hetero cyclic group R is an imidazole nucleus, a pyrazole nucleus, a pyrimidine nucleus, a pyridazine nucleus, a pyrazine nucleus, a purine nucleus, an acridine nucleus, a triazole nucleus, an oxadiazolc nucleus, a tetrazole nucleus, an indazole nucleus, a benzotriazole nucleus, a benzopyridazine nucleus, a benzopyrimidine nucleus, a benzopyrazine nucleus or a naphthylizine nucleus.

6. A method according to claim 1, wherein the adsorbent is an aliphatic type adsorbent composed of an aliphatic nitrogen containing compound having a functional chain containing 3 to 50 chain-constituting atoms but having no nitrogen-containing heterocycle and aromatic amino group, said aliphatic nitrogen atom being that of a primary amino, secondary amino, tertiary amino, quatemary ammonium or imino and bound to an aliphatic carbon atom; and
a porous base material having pore size of 50 nm to 1 µm;
said aliphatic nitrogen containing compound being bound to said base material directly or through a spacer compound selected from:
NH₂(CH₂)ₙNH₂,
NH₂(CH₂)ₙCOOH,
NH₂(CH₂)ₙOH
and
HOOC(CH₂)ₙCOOH
wherein n is an integer of 1 to 12.

7. A method according to claim 1, wherein said step of filtering non-adsorbed material through the filtration membrane is carried out by centrifuging the filtration unit.

8. A method according to claim 1, wherein the specimen is brought into contact with the adsorbent for at least 15 minutes at a temperature of 4 to 40°C, at a pH 4 to 8, and at an ionic strength of not higher than 0,2.

9. A method according to claim 2, wherein the reaction is carried out at a temperature of 25 to 40°C for 10 to 120 minutes.

10. A method according to claim 2, wherein the synthetic substrate is Ac-Ile-Glu-Ala-Arg-pNa, Boc-Leu-Gly-Arg-pNa or Boc-Leu-Gly-Arg-MCA, wherein Ac is acetyl, Boc is t-butoxycarbonyl, pNa is p-nitroaniline and MCA is 7-amino-4-methylcumarin.

## Patentansprüche

1. Ein Verfahren zur Bestimmung des Pyrogengehalts einer flüssigen Probe, bei der eine Filtriereinheit verwendet wird, die einen Filtratbecher und einen Probenbecher, welcher entfernbar am offenen Teil des Filtratbechers befestigt ist, wobei der genannte Probenbecher mit dem Filtratbecher über eine Filtrationsmembram verbunden ist, und wobei das Verfahren die Schritte einschließt:
die flüssige Probe in dem Probenbecher mit einem festen Adsorptionsmittel in Berührung bringen, welches in der Lage ist, selektiv Pyrogen zu adsorbieren,
das nichtadsorbierte Material durch die Filtriermembran unter solchen Bedingungen, daß die Permeation durch die Membran beschleunigt wird, filtrieren, um das nicht adsorbierte Material in den Filtratbecher wegzutransportieren und
das an dem in dem Probenbecher zurückgehaltenen Adsorptionsmittel adsorbierten Pyrgon mit einem Nachweismittel zur Reaktion bringen, um seinen Pyrogengehalt zu bestimmen.

2. Ein Verfahren gemäß Anspruch 1, bei dem nach dem Abschluß der Reaktion mit Limulus-Lysat als Nachweisreagens in Gegenwart einen synthetischen Substrats das Adsorptionsmittel von der Reaktionslösung durch die Filtriermembran mittels Zentrifugierens getrennt wird, um die Reaktionslösung in den Filtratbecher zu überführen und den Pyrogengehalt der Reaktionslösung zu bestimmen.

3. Ein Verfahren gemäß Anspruch 1, worin die Filtriermembran eine Ultrafiltriermembran mit einem Trenngrenzenmolekulargewicht von 10 000 bis 3 000 000 oder eine Mikrofiltriermembran mit einer Porenöffnung von 0,01 bis 10 µm ist.

4. Ein Verfahren gemäß Anspruch 1, bei dem das Adsorptionsmittel ein Adsorptionsmittel vom heterocyclischen Typ ist, welches aus einer stickstoffhaltigen cyclischen Verbindung der Formel
R-A-X
besteht, worin R eine stickstoffhaltige heterocyclische Gruppe ist, A eine Einfachbindung, eine Alkylengruppe mit 1 bis 12 Kohlenstoffatomen oder eine Alkenylengruppe mit 2 bis 12 Kohlenstoffatomen ist und X Wasserstoff, Amino, Hydroxy oder Carboxyl, wenn A eine Einfachbindung ist oder X Amino, Hydroxy oder Carboxyl ist, wenn A eine Alkylen- oder Alkenylengruppe ist, wobei die genannte heterocyclische Gruppe und die Alkylengruppe wahlweise mit einem oder mehreren Gruppen substituiert sind, welche aus Carboxyl, Oxo, einer Alkylgruppe mit 1 bis 6 Kohlenstoffatomen, Hydroxy, Amino und einer Alkoxygruppe mit 1 bis 6 Kohlenstoffatomen ausgewählt sind, und
einem wasserunlöslichen Träger besteht, welcher Hydroxy, Aminocarboxyl oder ein Halogenatom aufweist,
wobei die stickstoffhaltige cyclische Verbindung mit dem wasserunlöslichen Träger direkt oder über eine Spacerverbindung verbunden ist, welche aus:
NH₂(CH₂)ₙNH₂,
NH₂(CH₂)ₙCOOH,
NH₂(CH₂)ₙOH
und
HOOC(CH₂)ₙCOOH
ausgewählt ist, worin n eine ganze Zahlen zwischen 1 und 12 ist.

5. Ein Verfahren gemäß Anspruch 4, worin die stickstoffhaltige heterocyclische Gruppe ein Imidazolkern, ein Pyrazolkern, Pyrimidinkern, ein Pyridazinkern, ein Pyrazinkern, ein Purinkern, ein Acridinkern, ein Triazolkern, ein Oxadiazolkern, ein Tetrazolkern, ein Indazolkern, ein Benzotriazolkern, ein Benzopyridazinkern, ein Benzopyrimidinkern, ein Benzopyrazinkern oder ein Naphthylizinkern ist.

6. Ein Verfahren gemäß Anspruch 1, worin das Adsorptionsmittel ein Adsorptionsmittel vom aliphatischen Typ ist, welches aus einer aliphatischen Stickstoff enthaltenden Verbindung besteht, welche eine funktionelle Kette 3 bis 50 kettenbildenden Atomen aber keine stickstoffhaltige heterocyclische oder aromatische Aminogruppe enthält, worin das genannte aliphatische Stickstoffatom dasjenige aus primärem Amino, sekundärem Amino, teriärem Amino, quaternärem Ammonium oder Imino ist und an ein aliphatisches Kohlenstoffatom gebunden ist, und
einem porösen Grundmaterial besteht, welches eine Porengröße von 50 nm bis 1 µm hat,
wobei die genannte aliphatischen Stickstoff enthaltende Verbindung an dieses Grundmaterial direkt oder über eine Spacerverbindung gebunden ist, welche aus
NH₂(CH₂)ₙNH₂,
NH₂(CH₂)ₙCOOH,
NH₂(CH₂)ₙOH
und
HOOC(CH₂)ₙCOOH
ausgewählt ist, worin n eine ganze Zahl zwischen 1 und 12 ist.

7. Ein Verfahren gemäß Anspruch 1, worin der genannte Schritt des Abfilterns des nicht adsorbierten Materials durch die Filtriermembran durchgeführt wird, in dem die Filtriereinheit zentrifugiert wird.

8. Ein Verfahren gemäß Anspruch 1, bei dem die Probe mit dem Adsorptionsmittel mindestens 15 Minuten lang bei einer Temperatur von 4 bis 40 °C, einem pH von 4 bis 8 und einer Ionenstärke von nicht höher als 0,2 in Kontakt gebracht wird.

9. Ein Verfahren gemäß Anspruch 2, bei dem die Reaktion bei einer Temperatur von 25 bis 40 °C 10 bis 120 Minuten lang durchgeführt wird.

10. Ein Verfahren gemäß Anspruch 2, bei dem das synthetische Substrat Ac-Ile-Glu-Ala-Arg-pNa, Boc-Leu-Gly-Arg-pNa oder Boc-Leu-Gly-Arg-MCA ist, wobei Ac Acetyl ist, Boc t-Butoxycarbonyl ist, pNa ein p-Nitranilin ist und MCA 7-Amino-4-methylcumarin ist.

## Revendications

1. Procédé pour la détermination de la teneur en pyrogène d'un échantillon liquide utilisant une unité de filtration comprenant un vase de filtrat et un vase d'échantillon pouvant être fixé de manière détachable à la partie d'ouverture du vase de filtrat, ledit vase d'échantillon communiquant avec le vase de filtrat par l'intermédiaire d'une membrane de filtration, ledit procédé comprenant les étapes consistant :
à mettre l'échantillon liquide dans le vase d'échantillon en contact avec un adsorbant solide capable d'adsorber sélectivement le pyrogène,
à filtrer le matériau non adsorbé à travers la membrane de filtration dans des conditions pour accélérer la perméation à travers la membrane pour éliminer le matériau non adsorbé dans le vase de filtrat, et
à faire réagir le pyrogène adsorbé sur l'adsorbant retenu dans le vase d'échantillon avec un réactif de détection pour déterminer sa teneur en pyrogène.

2. Procédé selon la revendication 1, dans lequel, après achèvement d'une réaction avec un lysat de Limulus en tant que réactif de détection en présence d'un substrat synthétique, on sépare l'adsorbant de la solution de réaction par l'intermédiaire de la membrane de filtration par centrifugation pour transférer la solution de réaction dans le vase de filtrat et on détermine la teneur en pyrogène de la solution de réaction.

3. Procédé selon la revendication 1, dans lequel la membrane de filtration est une membrane d'ultrafiltration présentant un poids moléculaire d'arrêt (cutoff) de 10000 à 3000000 ou une membrane de microfiltration présentant une ouverture de pores de 0,01 à 10 µm.

4. Procédé selon la revendication 1, dans lequel l'adsorbant est un adsorbant de type hétérocyclique constitué d'un composé cyclique azoté de formule :
R-A-X
dans laquelle R est un groupe hétérocyclique azoté, A est une liaison simple, un groupe alkylène en C₁-C₁₂ ou un groupe alcénylène en C₂-C₁₂ et X est l'hydrogène, un groupe amino, hydroxy ou carboxyle, lorsque A est une liaison simple ou X est un groupe amino, hydroxy ou carboxyle, lorsque A est un groupe alkylène ou alcénylène ; ledit groupe hétérocyclique et le groupe alkylène étant facultativement substitués par un ou plusieurs groupes choisis parmi les groupes carboxyle, oxo, alkyle en C₁-C₆, hydroxy, amino et alcoxy en C₁-C₆ ; et
d'un support insoluble dans l'eau présentant un groupe hydroxy, amino, carboxyle ou un atome d'halogène ;
ledit composé cyclique azoté étant lié audit support insoluble dans l'eau directement ou par l'intermédiaire d'un composé d'espacement choisi parmi :
NH₂(CH₂)ₙNH₂,
NH₂(CH₂)ₙCOOH,
NH₂(CH₂)ₙOH
et
HOOC(CH₂)ₙCOOH
où n est un nombre entier de 1 à 12.

5. Procédé selon la revendication 4, dans lequel le groupe hétérocyclique azoté R est un noyau imidazole, un noyau pyrazole, un noyau pyrimidine, un noyau pyridazine, un noyau pyrazine, un noyau purine, un noyau acridine, un noyau triazole, un noyau oxadiazole, un noyau tétrazole, un noyau indazole, un noyau benzotriazole, un noyau benzopyridazine, un noyau benzopyrimidine, un noyau benzopyrazine ou un noyau naphtyridine.

6. Procédé selon la revendication 1, dans lequel l'adsorbant est un adsorbant de type aliphatique constitué d'un composé aliphatique azoté présentant une chaîne fonctionnelle contenant 3 à 50 atomes de constitution de chaîne mais ne présentant pas de groupe amino aromatique, ni d'hétérocycle azoté, ledit atome d'azote aliphatique étant celui d'un groupe amino primaire, amino secondaire, amino tertiaire, ammonium quatemaire ou imino et lié à un atome de carbone aliphatique ; et
d'un matériau de base poreux présentant une taille de pores de 50 nm à 1 µm ;
ledit composé aliphatique azoté étant lié audit matériau de base directement ou par l'intermédiaire d'un composé d'espacement choisi parmi :
NH₂(CH₂)ₙNH₂,
NH₂(CH₂)ₙCOOH,
NH₂(CH₂)ₙOH
et
HOOC(CH₂)ₙCOOH
où n est un nombre entier de 1 à 12.

7. Procédé selon la revendication 1, dans lequel on réalise ladite étape consistant à filtrer le matériau non adsorbé à travers la membrane de filtration par centrifugation de l'unité de filtration.

8. Procédé selon la revendication 1, dans lequel on met l'échantillon en contact avec l'adsorbant pendant au moins 15 min à une température de 4 à 40°C, à un pH de 4 à 8 et avec une force ionique qui n'est pas supérieure à 0,2.

9. Procédé selon la revendication 2, dans lequel on réalise la réaction à une température de 25 à 40°C pendant 10 à 120 min.

10. Procédé selon la revendication 2, dans lequel le substrat synthétique est Ac-Ile-Glu-Ala-Arg-pNa, Boc-Leu-Gly-Arg-pNa ou Boc-Leu-Gly-Arg-MCA, dans lequel Ac est l'acétyle, Boc est le t-butoxycarbonyle, pNa est la p-nitroaniline et MCA est la 7-amino-4-méthylcoumarine.
